# EUROPEAN PATENT SPECIFICATION

(11) **EP 0 600 200 B1**
(45) Date of publication and mention of the grant of the patent: **12.05.1999**
(21) Application number: 93116654.0
(22) Date of filing: 14.10.1993
(51) Int. Cl.: A61N 1/365

(54) **Detector for sensing events in living tissue**
Detektor zur Detektion von Ereignissen in lebendem Gewebe
Détecteur par détection des conditions dans un tissu vivant

(30) Priority: 03.12.1992 SE 9203642
(43) Date of publication of application: 08.06.1994
(60) Divisional application: 98120225.2
(73) Proprietor: Pacesetter AB, 171 95 Solna (SE)
(72) Inventor: Sivard, Ake, S-171 55 Solna (SE); Högnelid, Kurt, S-137 38 Västerhaninge (SE)
(74) Representative: Lettström, Richard Wilhelm

(56) References cited:
- EP-A- 0 321 764
- US-A- 4 766 902
- US-A- 5 050 599
- US-A- 5 103 819
- J.WEBSTER: "Medical Instrumentation and design: Electromyogram Integrators (pages 314-316)" , 1978, HOUGHTON MIFFLIN COMP., BOSTON

## Description

This invention relates to a detector for sensing events in living tissue according to the preamble of claim 1.

In US-A-4,041,953 is described a pacemaker which emits stimulation pulses to a heart when the heart does not beat spontaneously. In order to detect spontaneous events, the heart's electrical signals are sensed, amplified and sent to a level detector which senses whether the amplitude of the cardiac signal exceeds a level of detection. A spontaneous heart beat is deemed to have occurred if the amplitude exceeds the level of detection, and emission of a stimulation pulse is inhibited.

In US-A-4,708,144 is described a pacemaker which, in a similar manner, senses electrical signals from the heart and interprets the signal as spontaneous cardiac activity if the amplitude of the signal exceeds a level of detection. The level of detection is automatically adjusted through measurement of the peak value of the R wave in the cardiac signal, and a long-term average is calculated. The level of detection is then set according to the average value determined.

In Medical Instrumentation. Application and design; by J. Webster; Houghton Mifflin Comp. Boston, 1978:
"Electromyogram Integrators" , pages 314-316 is disclosed a detector for sensing events (EMG's), comprising a comparator for comparing an input signal with a reference, wherein the input signal is integrated over a predetermined time interval.

One problem with amplitude detectors of this kind is that the measurement signal often contains electrical noise from the surroundings. For the detector in the '953 pacemaker, the physician must program the pacemaker with a level of detection. This procedure is normally performed at a clinic in quiet, noise-free conditions. But after the patient leaves the clinic, the detector could interpret noise from electrically noisier surroundings as spontaneous heart beats. The pacemaker would then be inhibited when a stimulation pulse was actually necessary.

This problem is partially solved by automatic setting of the level of detection, as in the '144 pacemaker. The relationship between the level of detection and the average value for peak values for R waves results in some adaptation of the signal, picked up by the electrode, containing the noise. The averaging of peak values takes place over a relatively long period of time, i.e. for at least several minutes. As a result, adaptation takes a few minutes after any change from a noise-free to a noisy environment. During this period, the noise will have time to be interpreted as spontaneous heart beats, or some spontaneous heart beats could be missed. In addition, the noise must have a frequency different from the frequency of the heart's spontaneous heart rate if the level of detection is to be able to effectively compensate for that noise, i.e. measurement of the R wave will not help if noise has the same frequency as the heart rate.

Another problem which neither of the two detectors can solve in respect to heart signals concerns far-field signals, i.e. an event (spontaneous or stimulated) in the ventricle can be sensed in the atrium with an amplitude equal to the amplitude of a true event in the atrium. An ability to distinguish between signals with different origins would be useful in many situations.

An object of the invention is to achieve a detector which avoids the above problems and which is capable of reliably distinguish events in living tissue from noise.

Another object of the invention is to achieve a detector which automatically adapts the level of detection according to the level of noise.

Yet another object of the invention is to achieve a detector which automatically adapts expiration of a refractory period, in which the detector does not sense any events, to the prevailing noise and signal levels.

The first object is achieved with a detector according to claim 1.

The integration, which preferably lasts for an integration interval lasting 5 to 50 ms, yields the area of the tissue's electrical signals, instead of their amplitude, as a decision parameter. For brief periods of time, corresponding to 5 to 10 cardiac cycles, a spontaneous event generates, in principle, the same area, event after event. Noise contributes to the area with a value which is rather constant over two consecutive integration intervals. The effect of noise on the signal can then be easily identified as the events occur at long intervals, compared to the integration interval. A detector of this kind can advantageously be used with an amplitude detector to increase the reliability of decisions on whether or not an event has occurred.

The comparator also compares the input signal to a threshold value greater than the level of detection, an event then being deemed to have occurred if the input signal exceeds the level of detection without exceeding the threshold level during a defined first interval.

Since an interval is stipulated for the integrated area in which the input signal must be located for a given period of time for an event to be approved, severe, transient noise can easily be distinguished from events.

It is advantageous if the detector comprises averager which determines, from a defined number of events, the average value and peak value respectively of the input signal during a second defined interval after the input signal has passed the level of detection and a control device which sets the level of detection and/or threshold level on the basis of the average value.

In principle, the peak value after the input signal passes the level of detection corresponds to the maximum area in an event. So this determination is more independent of the noise frequency than the above-described prior art pacemaker in US-A-4,708,144. Here, the averager forms a normal value for the integral of the event and sets the detection and threshold levels on the basis thereof.

The detector can be devised so the control device sets the level of detection at a first defined part of the average value and the threshold value at the average value plus a second defined part of the average value.

Preferably, the detector further comprises a second integrator, which integrates the electrical signal over a longer integration interval than the first integrator, preferably 5 to 20 times longer, so as to generate a compensatory signal corresponding to prevailing noise superimposed on the electrical signals, said compensatory signal being sent to the control device in order to compensate the input signal or the level of detection for prevailing noise.

Since events (e.g. the heart's QRS wave) are chronologically very brief compared to inter-event periods, and noise can be regarded as relatively constant over brief periods of time, integration over 50 to 1000 ms produces a value for the prevailing level of noise. When the period of integration is in the latter part of the interval, the nadir value for the last second(s) should be used as take value for the prevailing level of noise. This value is employed as the compensatory signal for either direct compensation of the input signal or for control of the level of detection.

Here, it is an advantage if the control device sets the level of detection and/or threshold value according to the average value and the compensatory signal.

The detector advantageously comprises a rectifier which rectifies the electrical signals before they are integrated. Since the electrical signals generated by an event can have positive or negative polarity, the signals are integrated to prevent the two polarities from cancelling out one another in calculation of the integral.

Alternately, the first integrator integrates electrical signals with a first polarity, and the detector further contains an additional integrator which integrates electrical signals with a second polarity opposite to the first polarity.

In this manner, the detector can easily distinguish between signals with only one polarity (positive or negative) and signals with two polarities (positive and negative) on the basis of output signals from the first integrator and the additional integrator. Signals which e.g. originate in the atrium of a heart are detected as biphasic in the atrium and monophasic in the ventricle and vice-versa. So the detector can easily determine whether a signal originates in the atrium or the ventricle. Changing the sign of the output signal from one of the integrators and adding the two output signals produces the same result as if the signal had been rectified and integrated in an integrator.

The integrated electrical signals may be emitted by the integrators at intervals shorter than the integration interval.

This results in a floating integral value in which e.g. an integral value for the last 20 ms can be emitted every fifth ms. This would speed up the detector and compensate for the delay caused by integration.

To prevent double detection during an event, it would be an advantage if the detector inhibited the sensing of events during a defined refractory period after each sensed event. This normally takes place even after an emitted stimulation pulse.

Here it would be an advantage if the end of the refractory period were set at a third defined interval after the input signal drops below the level or detection following a detected event.

Alternately, the refractory period can be set to expire when the input signal drops below the compensatory signal.

The invention will be described below, referring to five figures, in which
FIG. 1 shows a pacemaker in which the detector according to the invention can be used to sense spontaneous heart beats;
FIG. 2 is a block diagram showing a first embodiment of the detector;
FIG. 3 is a diagram showing the conformation of the electrical signal in a spontaneous heart beat;
FIG. 4 is a diagram illustrating the function of the detector of FIG. 2 and
FIG. 5 is a block diagram showing a second embodiment of the detector.

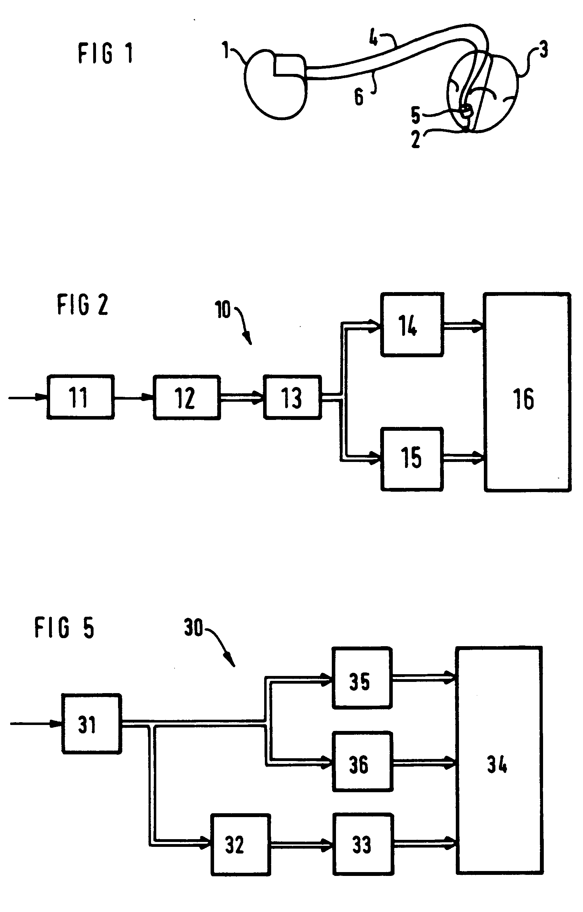

A pacemaker 1 in FIG. 1 is connected to a heart 3 by a tip electrode 2 in the ventricle of the heart 3 and a first electrode line 4 and by a ring electrode 5 with a second electrode line 6. The pacemaker 1 generates and emits stimulation pulses to the heart 3 in the absence of spontaneous cardiac activity.

In order to detect spontaneous heart activity, the pacemaker 1 contains a detector 10 as shown in FIG. 2. The detector 10 contains a filter amplifier 11 which amplifies and filters electrical signals from the heart 3. The filtered and amplified signals from the heart 3 are then digitized in an A/D converter 12 and sent to a rectifier 13. The rectified signal is then sent to a first integrator 14 which, after the signal has been digitized, functions as an adder which adds the digitized signal over a first integration interval, e.g. 20 ms, and to a second integrator 15 which integrates the signal over a longer integration period, e.g. 200 ms. The integrated signals are then sent to a microprocessor 16 at intervals of 5 ms and 100 ms respectively. In the microprocessor 16, the integrated signals are used for identifying spontaneous events. The microprocessor 16 performs software-instituted comparisons of the signal from the first integrator 14 and a detection level set by the two integrated signals. It also performs averaging etc. in a known manner.

The detector 10 can even be devised with analog circuits.

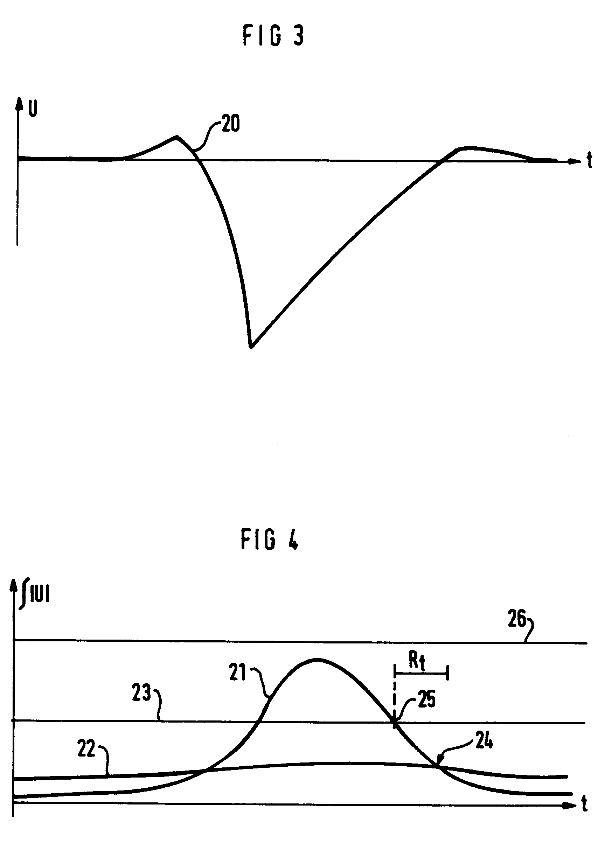

In FIG. 3 is shown what a heart signal 20 can look like when measured from the tip electrode 2 in the pacemaker 1. The large negative pulse is characteristic of the heart signal and is referred to as the R wave.

In FIG. 4 is shown the function of the detector 10 with a diagram in which the signal from the first integrator 14 is designated 21, and the signal from the second integrator is designated 22. The value of the briefly integrated signal 21 increases when an event occurs, then passing a detection level 23. It is accepted as a detected spontaneous event because it does not exceed a threshold value 26. The detection level 23 and the threshold level 26 are set on the basis of e.g. the signal with the long integration duration 22 which corresponds to the current level of noise. For example, the detection level can be set at half of the average value for peak values for the briefly integrated signal 21 in the last five events plus a standard nadir value for the signal with a long integration duration 22. The level of detection should not be allowed to drop below a defined value.

When the briefly integrated signal's 21 ascending flank passes the detection level 23, a refractory period starts during which the detector 10 is unable to approve additional events. When the descending flank of the briefly integrated signal 21 again passes the detection level 23, designated 25 in FIG. 4, a clock starts in the microprocessor which sets the refractory period at a defined time Rₜ, e.g. 25 ms.

Alternately, the end of the refractory period can be set to occur when the negative flank of the briefly integrated signal 21 passes the signal with a long integration duration 22, designated 24.

In addition, a refractory period, whose expiration can be set in one of the ways described above, starts after an emitted stimulation pulse.

In FIG. 5 is shown a second embodiment in which the detector 30 contains a signal conditioning unit 31 in which the electrical input signal is amplified, filtered and A/D-converted before being passed on. The output signal from the signal processing unit 31 is then sent on in three parallel signal channels. In the first channel, the signal is rectified in a rectifier 32 and then sent to a first integrator 33 in which the rectified signal is integrated over a long integration interval in the same way as in the detector 10 in FIG. 2. In the second channel, the signal is sent to a second integrator 35 which integrates the positive part of the signal over a brief integration interval. In the third channel, the signal is sent to a third integrator 36 which integrates the negative part of the signal over a brief integration interval. The sum of the absolute values for the output signals from the second integrator 35 and the third integrator 36 is the same as the output signal from the briefly integrating integrator 14 shown in FIG. 2. The integrated signals are sent to a microprocessor 34 in which detection levels and threshold levels can be set in the same way as in the microprocessor 16 shown in FIG. 2. Here, the microprocessor 34 can also distinguish between monophasic and biphasic signals. When a heart is sensed, this means that signals originating in the atrium can be identified from both the atrium and the ventricle, and signals originating in the ventricle can also be identified from the atrium and the ventricle.

## Claims

1. A detector (10) for sensing events in living tissue (3), comprising a comparator (16) which compares an input signal corresponding to electrical signals from the living tissue with a defined signal detection level, a first integrator (14) which generates the input signal (21) by integrating the electrical signals (20) from the tissue (3) over a defined integration interval, characterized in that the comparator (16) compares the input signal (21) with a threshold level (26) higher than the level of detection (23), the comparator (16) determining an event to have occurred if the input signal (21) exceeds the defined level of detection (23) without exceeding the threshold level (26) during a defined first interval.

2. A detector of claim 1, wherein an averager (16) determines, from a defined number of events, the average value and peak value respectively of the input signal (21) during a second defined interval after the input signal (21) has passed the level of detection (23) and a control device (16) which sets the level of detection (23) and/or threshold level (26) on the basis of the average value.

3. A detector of claim 2, wherein the control device (16) sets the level of detection (23) at a first defined part of the average value and the threshold value (26) at the average value plus a second defined part of the average value.

4. A detector of any of claims 1 - 3, wherein a second integrator (15) integrates the electrical signals (20) over a longer integration interval than the first integrator (14), preferably 5 to 20 times longer, so as to generate a compensatory signal (22) corresponding to prevailing noise superimposed on the electrical signals (20), said compensatory signal (20) being sent to the control device in order to compensate the input signal (21) or the level of detection (23) for prevailing noise.

5. A detector of claim 4, in combination with claim 3, wherein the control device (16) sets the level of detection (23) and/or threshold level (26) on the basis of the average value and the compensatory signal (22).

6. A detector of any of the above claims, wherein a rectifier (13) rectifies the electrical signals (20) before they are integrated.

7. A detector of claims 1 - 5, wherein the first integrator (14) integrates electrical signals with a first polarity, and the detector further contains an additional integrator which integrates electrical signals with a second polarity opposite to the first polarity.

8. A detector of any of the above claims, wherein the integrated electrical signals (21, 22) are emitted by the integrators (14, 15) at intervals shorter than the integration interval.

9. A detector of any of the above claims, wherein the detector inhibits sensing of events during a defined refractory period after each sensed event.

10. A detector of claim 9, wherein the end of the refractory period is set at a defined interval (Rₜ) after the input signal (21) drops below the level of detection (23) following a detected event.

11. A detector of claim 8, in combination with claim 4 or 5, wherein the refractory period expires when the input signal (21) drops below the compensatory signal (22).

## Patentansprüche

1. Ein Detektor (10) zum Abfühlen von Ereignissen in lebendem Gewebe (3) mit einem Komparator (16), der ein zu elektrischen Signalen von dem lebenden Gewebe korrespondierendes Eingangssignal mit einem definierten Signaldetektionsniveau vergleicht, einem ersten Integrator (14), der das Eingangssignal (21) durch Integrieren des elektrischen Signals (20) von dem Gewebe (3) über ein definiertes Integrationsintervall erzeugt, **dadurch gekennzeichnet,** daß der Komparator (16) das Eingangssignal (21) mit einem Schwellwert (26), der größer ist als das Detektionsniveau (23), vergleicht, wobei der Komparator (16) entscheidet, daß ein Ereignis eingetreten ist, wenn das Eingangssignal (21) das definierte Detektionsniveau (23) übersteigt, ohne den Schwellwert (26) wahrend eines definierten ersten Intervalls zu übersteigen.

2. Ein Detektor nach Anspruch 1, in dem ein Mittelwertbildner (16) aus einer bestimmten Anzahl von Ereignissen den Mittelwert beziehungsweise den Spitzenwert des Eingangssignals (21) während eines zweiten definierten Intervalls, nachdem das Eingangssignal (21) das Detektionsniveau (23) passiert hat, bestimmt und mit einer Steuervorrichtung (16), die das Detektionsniveau (23) und/oder den Schwellwert (26) auf der Basis des Mittelwertes einstellt.

3. Ein Detektor nach Anspruch 2, worin die Steuervorrichtung (16) das Detektionsniveau (23) auf einen ersten definierten Teil des Mittelwertes und den Schwellwert (26) auf den Mittelwert plus einem zweiten definierten Teil des Mittelwertes einstellt.

4. Ein Detektor nach einem der Ansprüche 1 - 3, worin ein zweiter Integrator (15) das elektrische Signal (20) über ein längeres Integrationsintervall als der erste integrator (14), vorzugsweise 5 bis 20 mal länger integriert, um ein zu dem aktuellen, dem elektrischen Signal überlagerten Störsignal korrespondierendes Kompensationssignal (22) zu erzeugen, wobei das Kompensationssignal (20) zu der Steuervorrichtung gesendet wird, um das Eingangssignal (21) oder das Detektionsniveau (23) hinsichtlich des aktuellen Störsignals zu kompensieren.

5. Ein Detektor nach Anspruch 4 in Kombination mit Anspruch 3, worin die Steuervorrichtung (16) das Detektionsniveau (23) und/oder den Schwellwert (26) auf der Basis des Mittelwertes und des Kompensationssignals (22) einstellt.

6. Ein Detektor nach einem der vorhergehenden Ansprüche, worin ein Gleichrichter (13) die elektrischen Signale (20) gleichrichtet, bevor sie integriert werden.

7. Ein Detektor nach den Ansprüchen 1 - 5, worin der erste Integrator (14) die Signale mit einer ersten Polarität integriert und der Detektor weiterhin einen zusätzlichen Integrator aufweist, der die elektrischen Signale mit einer zweiten, zu der ersten entgegengesetzten Polarität integriert.

8. Ein Detektor nach einem der vorhergehenden Ansprüche, worin die integrierten elektrischen Signale (21, 22) von den Integratoren (14, 15) in Intervallen abgegeben werden, die kürzer sind als das Integrationsintervall.

9. Ein Detektor nach einem vorhergehenden Ansprüche, worin der Detektor das Abfühlen von Ereignissen während einer definierten Refraktärzeit nach jedem abgefühlten Ereignis inhibiert.

10. Ein Detektor nach Anspruch 9, worin das Ende der Refraktärzeit auf ein definiertes Intervall (Rₜ), nachdem das Eingangssignal (21) nach einem detektierten Ereignis unter das Detektionsniveau (23) fällt, eingestellt wird.

11. Ein Detektor nach Anspruch 8 in Kombination mit den Ansprüchen 4 und 5, worin die Refraktärzeit abläuft, wenn das Eingangssignal (21) unter das Kompensationssignal (22) fällt.

## Revendications

1. Capteur (10) pour détecter des événements dans des tissus (3) vivants, comportant un comparateur (16) qui compare un signal d'entrée correspondant à des signaux électriques provenant du tissu vivant avec un niveau défini de détection de signal, un premier intégrateur (14) qui produit le signal (21) d'entrée en intégrant les signaux (20) électriques provenant du tissu (3) sur un intervalle d'intégration défini, caractérisé en ce que le comparateur (16) compare le signal (21) d'entrée à un niveau (26) de seuil supérieur au niveau de détection (23), le comparateur (16) déterminant un événement qui a eu lieu si le signal (21) d'entrée dépasse le niveau défini de détection (23) sans dépasser le niveau (26) de seuil pendant un premier intervalle défini.

2. Capteur suivant la revendication 1, dans lequel un dispositif (16) de formation de moyenne détermine, à partir d'un certain nombre défini d'événement, la valeur moyenne et la valeur de pic respectivement du signal (21) d'entrée pendant un second intervalle défini après que le signal (21) d'entrée a passé le niveau de détection (23) et un dispositif (16) de commande qui règle le niveau de détection (23) et/ou le niveau (26) de seuil sur la base de la valeur moyenne.

3. Capteur suivant la revendication 2, dans lequel le dispositif (16) de commande règle le niveau détection (23) à une première partie définie de la valeur moyenne et la valeur (26) de seuil à la valeur moyenne plus une seconde partie définie de la valeur moyenne.

4. Capteur suivant l'une quelconque des revendications 1 à 3, dans lequel un second intégrateur (15) intègre les signaux (20) électriques sur un intervalle d'intégration plus long que le premier intégrateur (14), de préférence 5 à 20 fois plus long, de manière à produire un signal (22) de compensation correspondant au bruit qui prévaut et qui est superposé aux signaux (20) électriques, le signal (20) de compensation étant envoyé au dispositif de commande afin de compenser le signal (21) d'entrée ou le niveau de détection (23) pour le bruit qui prévaut.

5. Capteur suivant la revendication 4 en combinaison avec la revendication 3, dans lequel le dispositif (16) de commande règle le niveau de détection (23) et/ou le niveau (26) de seuil sur la base de la valeur moyenne et du signal (22) de compensation.

6. Capteur suivant l'une quelconque des revendications précédentes dans lequel un redresseur (13) redresse les signaux (20) électriques avant qu'ils ne soient intégrés.

7. Capteur suivant l'une des revendications 1 à 5, dans lequel le premier intégrateur (14) intègre des signaux électriques avec une première polarité, et le capteur comporte en outre un intégrateur supplémentaire qui intègre des signaux électriques avec une seconde polarité opposée à la première polarité.

8. Capteur suivant l'une quelconque des revendications précédentes, dans lequel les signaux (21,22) électriques intégrés sont émis par les intégrateurs (14,15) à des intervalles plus courts que l'intervalle d'intégration.

9. Capteur suivant l'une quelconque des revendications précédentes, dans lequel le capteur empêche la détection des événements pendant une période réfractaire définie après chaque événement détecté.

10. Capteur suivant la revendication 9, dans lequel la fin de la période réfractaire est réglée à un intervalle (Rₜ) défini après que le signal (21) d'entrée soit tombé en dessous du niveau de détection (23) à la suite d'un événement détecté.

11. Capteur suivant la revendication 8, en combinaison avec la revendication 4 ou 5, dans lequel la période réfractaire expire lorsque le signal (21) d'entrée tombe en-dessous du signal (22) de compensation.
